# EUROPEAN PATENT SPECIFICATION

(11) **EP 0 536 083 B1**
(45) Date of publication and mention of the grant of the patent: **06.03.1996**
(21) Application number: 92810723.4
(22) Date of filing: 22.09.1992
(51) Int. Cl.: C07C 69/757, C07C 67/343, C07C 229/48

(54) **Process for the synthesis of dialkyl succinylsuccinate esters and their conversion to dialkyl 2,5-diarylamino-3,6-dihydroterephthalate esters**
Verfahren zur Synthese von Succinylobernsternsäuredialkylestern und ihre Umwandlung in Dialkyl 2,5-Diarylamino-3,6-Dihydroterephthalsäureester
Procédé de synthèse de dialkyle succinylsuccinates et leur conversion en esters 2,5-diarylamino-3,6-dihydrotéréphthaliques

(30) Priority: 30.09.1991 US 769991; 02.07.1992 US 907896
(43) Date of publication of application: 07.04.1993
(73) Proprietor: CIBA-GEIGY AG, CH-4002 Basel (CH)
(72) Inventor: Campbell, Colin D., Dr., Wilmington, DE 19803 (US); Jaffe, Edward E., Dr., Wilmington, DE 19803 (US)

(56) References cited:
- EP-A- 0 057 873
- EP-A- 0 128 489

## Description

Processes for the preparation of dialkyl succinylsuccinates from dialkyl succinates and alkali metal alcoholates in an organic solvent are well known. Such compounds are intermediates in the preparation of quinacridone pigments. Dialkyl succinates usually have been exemplified by the readily available dimethyl and diethyl esters, but higher alkyl groups such as iso-propyl (EP 128489) and n-butyl (JP 82-183744) have also been utilized. The alkali metal alcoholates employed are usually the sodium or potassium salts of the alcohols corresponding to the alkyl group in the dialkyl succinate, but mixed systems are known (e.g. methyl-isopropyl in EP 128489). The alkali metal alcoholates are either prepared in situ from the alkali metal and the alcohol or used as commercially available reagents. In either instance, the alcohol usually is used in excess to act as a cosolvent. Solvents for such processes have included dioxan (US 2,803,644), decalin (US 2,782,220), Dowtherm A (a mixture of 73.5% diphenyl ether and 26.5% biphenyl; US 2,821,541, US 3,024,268), xylene (JP 77-59135), dimethylformamide (US 3,045,040) and methanol (US 4,435,589).

Process improvements in terms of yield have been alleged as the result of the addition to the reaction mixture of sulfur compounds such as dimethylsulfoxide (JP 77-59135, JP 69-27577), polyalkylene glycols, alkylene glycol alkyl esters or polyalkylene glycol alkyl ethers (JP 86-286343), phosphoric acid amides, phosphoric acid esters and/or cyclic N-alkyl amides (JP 87-106062). In all of these processes, the initially formed alkali metal salt of the dialkyl succinylsuccinate is hydrolyzed to the dialkyl succinylsuccinate with acid.

While many of these processes claim yields of dialkyl succinylsuccinates in the area of 80%, such yields are generally achieved at the expense of increasing cost of reactants and/or adjuvants (EP 128489, JP 86-286343, JP 87-106062). The use of an all methyl system results in less soluble esters, thereby limiting the throughput when translated to a commercial scale. Additionally, lower yields of dimethylsuccinylsuccinate are generally obtained unless a co-solvent (JP 77-59135) or pressure (U.S. 4,435,589) is used.

It has been surprisingly determined that these shortcomings can be overcome by the separate or in-situ transesterification of the most readily available and economical dialkyl succinate, i.e. dimethyl succinate, with aliphatic alcohols having two to six carbon atoms and conducting the condensation of the resulting mixed dialkyl succinates to the disodium salt of mixed dialkyl esters of succinylsuccinic acid with alkali metal alcoholates in organic solvents. The use of these reactants together with the appropriate choice of reaction conditions results in yields of over 80% of theory of mixed dialkyl succinylsuccinates (after acidification). These esters additionally exhibit enhanced solubility in organic solvents relative to the pure dimethyl succinylsuccinates and greatly increased solubility in organic solvents upon conversion to mixed dialkyl 2,5-diarylamino-3,6-dihydroterephthalic esters. Finally, the mixed dialkyl 2,5-diarylamino-3,6-dihydroterephthalic esters can be converted into dihydroquinacridones by processes known in the art (e.g., US 2,821,529, 3,009,916) wherein the enhanced solubility of the intermediate mixed esters allows for greater throughput to be achieved. The dihydroquinacridones are available for oxidation to red and violet quinacridone pigments of value in the paint, ink and plastics industries.

The process of the instant invention is conducted by transesterification of dimethyl succinate using ethanol or isobutanol, preferably ethanol, in the presence of a transesterification catalyst. The process may be carried out by using the aliphatic alcohol as the solvent or by including an additional organic solvent.

Additional organic solvents include aromatic solvents such as toluene and xylene; diphenyl ether; mixtures of alkylated diphenyl ethers, and mixtures of diphenyl ether or alkylated diphenyl ethers and biphenyl or alkylated biphenyls (for example, Dowtherm A (diphenyl ether 73.5% and biphenyl 26.5%). The transesterification catalyst may be any of the catalysts well known in the art but is preferably an alkaline metal alcoholate such as sodium methylate, which is itself transformed into a mixture of sodium methylate and sodium alcoholate during the process of transesterification with the alcohol.

The amount of catalyst ranges from 0.01 to 5%, by weight of dimethyl succinate, and preferably 0.1 to 2%. The alcohol will be present in an amount of 1 to 10 moles per mole of dimethyl succinate and preferably 2 to 5 moles.

The transesterification reaction is conducted at ambient temperatures to 100°C, with or without additional solvent, and proceeds further if the methanol formed during the reaction is removed by (a) applying vacuum at ambient temperature, (b) heating the mixture so that the methanol distills out of the reaction mixture or (c) a combination of these two procedures. The mixed esters of succinic acid so produced may be isolated by conventional means or, preferably, used directly to prepare the corresponding succinylsuccinate esters.

The reaction may be monitored by methods capable of separating the product ester (dimethyl, methyl-alkyl, dialkyl succinates) and the relative proportions can be controlled by cooling or by recycling the methanol. Methods of analysis include gas liquid chromatography and high performance liquid chromatography.

Conversion of the transesterified dialkyl succinate mixture to dialkyl succinylsuccinate mixture (H₂DASS) proceeds according to the following equation:

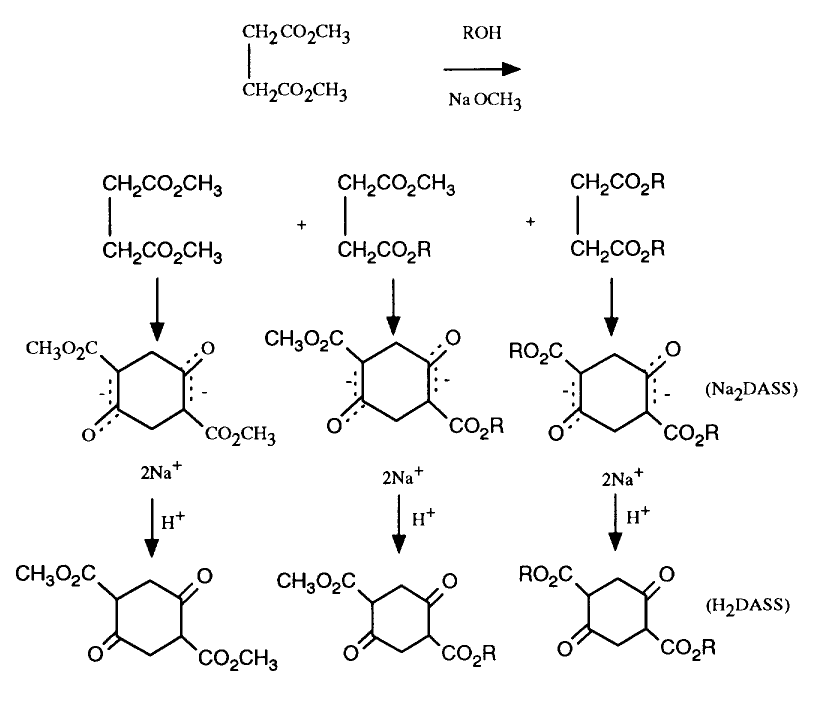

The condensation of two molecules of dialkyl succinate to one molecule of the disodium salt of dialkyl succinylsuccinate (Na₂DASS) in the presence of at least two molecules of sodium alcoholate proceeds in yields of up to 85% and has been previously described (eg., US 3,024,268). The reaction proceeds more quickly and in higher yield when the final temperature of the heterogeneous mixture of Na₂DASS is 100-160°C, preferably 120-140°C.

Quantities of reagents (per mole of dialkyl succinate) include from 1 to 3 moles sodium alkylate, and preferably 1 to 1.5 moles; and 1 to 10 moles alcohol and preferably 2 to 5 moles, for solubilizing the sodium alcoholate in the solvent. The amount of extraneous solvent is chosen such that the final dialkyl succinylsuccinate product forms a 10-50% by weight solution, preferably 20-40% in the solvent.

The Na₂DASS salt mixture may be isolated by filtration and washing to eliminate solvent and by-products or may be converted directly to H₂DASS by reaction with aqueous acid (eg., acetic acid, sulfuric acid, hydrochloric acid) or non-aqueous acid (eg., glacial acetic acid, formic acid) in an amount capable of neutralizing all of the sodium salts (Na₂DASS and excess sodium alkylate).

The H₂DASS so obtained, either in solid form or in solution, may be converted to dialkyl 2,5-diarylamino-3,6-dihydroterephthalic esters by condensation with an excess of arylamine (e.g. aniline) by methods well known in the art (eg., US 2,821,541, US 3,372,184) and such disclosures are fully incorporated herein. The enhanced solubility of the H₂DASS ester mixture permits the condensation reaction to be conducted at higher concentrations (at equal temperature) than the applicable concentration when utilizing single esters. After removal of excess of arylamine and condensing catalyst (e.g. trifluoroacetic acid), highly concentrated solutions of dialkyl 2,5-diarylamino-3,6-dihydroterephthalic esters in the solvent are obtained. These concentrated solutions allow greater throughput of these intermediates and correspondingly increased production of quinacridone pigments.

The process according to the invention yield high purity dialkyl 2,5-diarylamino-3,6-dihydroterephthalic esters in high yields in concentrated solution. These solutions can be reacted further to give dihydroquinacridone (e.g. US 3,009,916) from which quinacridone can be obtained by oxidation (e.g. US 2,969,399 and US 3,287,457).

The following examples further illustrate the preferred embodiments of this invention. In these examples, are parts and percentages are by weight unless otherwise noted.

Example 1: A reaction vessel is charged with anhydrous ethanol (138 g, 3 mole) and sodium methylate (1.0 g, 0.0185 mole) and the mixture stirred under nitrogen until homogeneous. Dimethyl succinate (146 g, 1 mole) is added and the mixture maintained at 25°C for 30 minutes. Analysis of the mixture after filtration identifies a succinate ester ratio of dimethyl 19.6%, methyl ethyl 49.9% and diethyl 30.5%. Concentration of succinates in the mixture is 58.3%.

Example 2: A solution of sodium methylate (1.0 g, 0.0185 mole) in anhydrous ethanol (138 g, 3 mole) is admixed with dimethyl succinate (146 g, 1 mole) at 25°C. The alcohols are removed almost completely by stripping at 3.3·10³ Pa at 25°C for two hours. Analysis of the mixture after filtration identifies a succinate ester ratio of dimethyl 10.4%, methyl ethyl 44.2% and diethyl 45.4%. Concentration of succinates in the mixture is 97.4%.

Example 3: A mixture of anhydrous isobutanol (222 g, 3 mole), sodium methylate (0.54 g, 0.01 mole) and dimethyl succinate (146 g, 1 mole) is analyzed immediately after mixing at room temperature (25°C). Succinate ratios are dimethyl 16.0%, methyl isobutyl 43.1%, di-isobutyl 40.9%.

The mixture is heated to 100°C and the alcohols collected by distillation. After three hours, the product is filtered and analyzed for succinate ratio:dimethyl 4.6%, methyl isobutyl 37.6%, di-isobutyl 57.8%.

### General Process for Preparation of Dialkyl Succinylsuccinate Esters

Example 4: ®Dowtherm A (100 g, 0.6 mole; eutectic mixture of 26.5 % biphenyl and 73,5 % diphenyl ether) is mixed with dry sodium methylate (64.8 g, 1.2 mole) and to this well stirred slurry at 25°C under nitrogen is added the identified anhydrous alcohol (3.5 moles). The mildly exothermic reaction is allowed to continue until the sodium methylate had largely gone into solution. Dialkyl succinate (1 mole) is added steadily at temperature below 50°C, and on completion, the mixture is gradually heated with efficient stirring. Alcohols are distilled at ambient pressure and fluidity of the heterogeneous reaction maintained by gradual addition of ®Dowtherm A during the distillation. Termination of the reaction is assessed by a final temperature of 140°C or by analysis of the filtrate of a filtered sample for residual succinates (less than 2% of input).

On completion, the slurry of disodium succinylsuccinate ester is cooled to 70°C, treated with 45% aqueous acetic acid (containing 1.25 moles acetic acid) at 70°C and stirred until all solids are in solution whereupon the layers are separated at 70°C. The upper ®Dowtherm A layer is twice extracted at 70°C with 22% aqueous sodium chloride solution, dried by dewatering at 90°C / 3.3·10³ Pa pressure and filtered to remove residual salt. The clear, pale yellow solution of succinylsuccinate ester in ®Dowtherm A at 70°C can be used directly in the production of dialkyl 2,5-diarylamino-3,6-terephthalic esters. Alternatively, the mixed succinylsuccinate esters which precipitate as crystalline materials on cooling of the solution can be isolated by filtration, followed by washing ®Dowtherm A-free with a suitable solvent and drying.

Example 5 (comparative): Upon using methanol and dimethyl succinate in the process of Example 4, dimethyl succinylsuccinate in an 61% yield is obtained. It exhibits solubility in ®Dowtherm A of about 10% at 75°C to 50% at 125°C.

Example 6: Upon using ethanol and dimethyl succinate in the process of Example 4, a product with an ester ratio of dimethyl 35%, methyl ethyl 49%, diethyl 16% is obtained in 80% yield. This mixed ester exhibits an increase in solubility in ®Dowtherm A over the all-methyl system as evidenced by a 40% solubility at 75°C and 60% solubility at 90°C.

Example 7: Upon using ethanol and the transesterified succinate ester of Example 1 in the process of Example 4, a product with an ester ratio of dimethyl 25% methyl ethyl 50%, diethyl 25% is obtained in 78% yield, which product exhibits greater solubility in ®Dowtherm A than that of Comparative Example 5.

Example 8: Upon using ethanol and the transesterified succinate ester of Example 2 in the process of Example 4, a product with an ester ratio of dimethyl 4.3%, methyl ethyl 32.7%, diethyl 63.0% is obtained in 81% yield which product exhibits greater solubility in ®Dowtherm A than that of Comparative Example 5.

Example 9: Upon using isobutanol and the transesterified succinate ester of Example 3 in the process of Example 4, a product is obtained with an ester ratio of dimethyl 6.0%, methyl isobutyl 35.0%, di-isobutyl 59.0% in 75% yield, which product exhibits greater solubility in ®Dowtherm A than that of Comparative Example 5.

### Pre-transesterification of dimethylsuccinate with ethanol followed by conversion to succinylsuccinate ester

Example 10: A vessel is charged with anhydrous ethanol (161 g, 3.5 mole) and sodium methylate (1.0 g, 0.0185 mole) and stirred at 25°C to affect solution. Dimethyl succinate (146 g, 1 mole) is added, and the mixture heated to 80°C when alcohols first start to distill. The temperature is gradually increased to 120°C with collection of alcohols distilling in the range 72-76°C. The mixture is cooled to 60°C, whereupon ®Dowtherm A (92 ml) is added followed by anhydrous ethanol (100 ml) and, at 40°C, sodium methylate (64.8 g, 1.2 mole). This mixture, which becomes homogeneous at 55-60°C, is heated slowly with efficient stirring and with continuous removal of alcohols to 125°C over 4 hours. During this period, ^{®}Dowtherm A (128 ml) is slowly added. The resulting dark mass is cooled to 70°C and neutralized with 45% aqueous acetic acid (165 ml). The ®Dowtherm A layer is separated, washed twice with 22% aqueous sodium chloride solution, dewatered at 90°C and 3.3·10³ Pa pressure, and filtered to give an 80% yield of mixed succinylsuccinates (dimethyl 15%, methyl ethyl 47%, diethyl 38%) at 30% concentration.

### In-situ transesterification of dimethyl succinate with ethanol

Example 11: A vessel is charged with ®Dowtherm A (92 ml) and sodium methylate (64.8 g, 1.2 mole) and anhydrous ethanol (200 ml) is added to this well-stirred slurry under nitrogen. A temperature rise to 62°C is noted. On cooling to 50°C, dimethyl succinate is added (146 g, 1 mole) over ten minutes whereupon the mixture becomes homogeneous. Heating is applied and the sodium salt of mixed succinylsuccinic esters starts to precipitate at temperatures above 60°C. Alcohols start to distill at 90-100°C together with some mixed ether by-products. Heating is continued with collection of alcohols (b.p. 70-75°C) by distillation and addition of ®Dowtherm A (115 ml) at a rate approximately equal to the rate of distillation. When the temperature of the reaction mass reaches 130°C (3 hours), the mixture is cooled to 70°C and 45% aqueous acetic acid (165 ml) is carefully added with stirring. When a total solution is formed, the lower dark aqueous layer (pH 6.4) is discarded and the upper ®Dowtherm A layer washed twice with 22% aqueous sodium chloride solution at 70°C. The organic phase is dewatered at 90°C / 3.3·10³ Pa and then filtered. Analysis shows a concentration of dialkyl succinylsuccinate esters in ®Dowtherm A of 30.5% with a yield of 81% theory. The ratio of esters in succinylsuccinates is dimethyl 28.2%, methyl ethyl 49.0% diethyl 22.8%. Residual succinates in ®Dowtherm A are 0.4%.

Example 12: The method of Example 11 is repeated with the exception that only 60 ml ®Dowtherm A are added during the distillation. Workup yields a product with a similar ester ratio but at a concentration of 37.5% succinylsuccinate esters in ®Dowtherm A.

### General process for preparation of dialkyl 2,5-diarylamino-3,6-dihydroterephthalic esters (DAT)

Example 13: A mixture of arylamine (2.25 moles) and trifluoroacetic acid (0.01 mole) in ®Dowtherm A (1.2 moles) at 50°C is admixed with a solution of mixed succinylsuccinate esters (1.0 mole) in ®Dowtherm A (2 to 4 moles) at 70-90°C under nitrogen. After holding at 90°C for 1 hour at 16.7·10³ Pa and distilling the water (2 moles) of condensation, the pressure is dropped to 1.3-2.6·10³ Pa and the temperature is increased to 120°C to distill out the excess amine and the amine-trifluoroacetate salt. Additional ®Dowtherm A is added during the later stages to maintain a suitable concentration (25-45%) of product. The vacuum is replaced by a nitrogen atmosphere on completion.

The dialkyl 2,5-diarylamino-3,6-dihydroterephthalic esters can be isolated by cooling the ®Dowtherm A solution, collection by filtration and washing with a suitable organic solvent to remove ®Dowtherm A (e.g. methanol or petroleum spirits). Alternatively, the solution of the product under nitrogen can be converted directly to the corresponding dihydroquinacridone by pyrolysis.

Example 14 (comparative): Dimethyl succinylsuccinate from Example 5 is reacted with aniline as described in Example 13 to give a 96% yield of dimethyl 2,5-dianilino-3,6-dihydroterephthalate. Its solubility profile in ®Dowtherm A ranges from 7.5% at 120°C to 50% at 190°C.

Example 15: Mixed methyl-ethyl 2,5-dianilino-3,6-dihydroterephthalates are prepared from the product of Example 6 with aniline by the process of Example 13 to obtain a 93% yield (isolated). The solubility is clearly far greater in ®Dowtherm A than that of the all methyl system as evidenced by 17% solubility at 120°C to 50% solubility at 165°C.

Example 16: The mixed ester system prepared in Example 7 is converted to the DAT ester with aniline by the process described in Example 13. The product is obtained in a 92% yield and has a solubility profile showing greatly enhanced solubility in ®Dowtherm A over that of comparative Example 14.

Example 17: In a similar manner, the mixed ester systems of Examples 8-12 are converted with aniline to the mixed ester DAT by the process of Example 13. All products exhibit greatly enhanced solubility in ®Dowtherm A over that of comparative Example 14.

Example 18: The mixed ester succinylsuccinate from Example 6 is converted to the mixed ester of 2,5-di-p-tolyl-3,6-dihydroterephthalic acid using p-toluidine in place of aniline in the process of Example 13. The product exhibits a solubility of 18% in ®Dowtherm A at 160°C as compared with a solubility of 9% for the corresponding dimethyl ester.

Summarizing, it is seen that this invention provides a new mixed ester process for the preparation of dialkyl succinylsuccinate esters. Variations may be made in proportions, procedures and materials without departing from the scope of the invention as defined by the following claims.

## Claims

1. A process for the preparation of mixed methyl/ethyl or methyl/isobutyl dialkyl succinylsuccinates which comprises the steps of (1) transesterifying dimethylsuccinate with ethanol or isobutanol in the presence of a transesterification catalyst; (2) condensing two moles of the mixed dialkyl succinate of step (1) to the mixed methyl/ethyl or methyl/isobutyl dialkyl succinylsuccinate or the disodium salt thereof at temperatures of 100-160°C in the presence of sodium C₁-C₆alkylate and alcohol; and (3) recovering the condensed product.

2. The process of claim 1, wherein a mixed methyl/ethyl dialkyl succinylsuccinate is prepared using ethanol in step (1).

3. The process of claim 1, wherein said catalyst is a metal alcoholate.

4. The process of claim 1, wherein step (1) occurs at ambient temperatures to 100°C.

5. The process of claim 1, wherein in step (1) said alkanol is present in an amount of 1 to 10 moles per mole of dimethyl succinate and said catalyst is present in an amount of 0.01-5%, by weight of dimethyl succinate.

6. The process of claim 1, wherein an organic solvent other than said alkanol is present during steps (1) and (2).

7. The process of claim 1, wherein in step (2) the sodium alkylate is present in an amount of 1 to 3 moles and the alcohol is present in an amount of 1 to 10 moles, each per mole of mixed dialkyl succinate.

8. The process of claim 1, wherein steps (1) and (2) are conducted in-situ.

9. A process for the preparation of methyl/ethyl or methyl/isobutyl dialkyl 2,5-diarylamino-3,6-dihydroterephthalate esters which comprises condensing the mixed methyl/ethyl or methyl/isobutyl dialkyl succinylsuccinate resulting from step (2) of the process of claim 1 with an excess of an arylamine.

10. The process of claim 6, wherein the additional solvent is a mixture of (a) diphenyl ether or one or more alkylated diphenyl ethers or a mixture thereof and (b) biphenyl or one or more alkylated biphenyls or a mixture thereof.

## Patentansprüche

1. Verfahren zur Herstellung von gemischten Methyl/ Ethyl- oder Methyl/Isobutyl-Dialkylsuccinylsuccinaten, umfassend die Schritte (1) Umesterung von Bernsteinsäuredimethylester mit Ethanol oder Isobutanol in Gegenwart eines Umesterungskatalysators; (2) Kondensieren von 2 Mol des gemischten Dialkylsuccinats von Schritt (1) zu dem gemischten Methyl/Ethyl- oder Methyl/Isobutyl-Dialkylsuccinylsuccinat oder dem Dinatriumsalz davon bei Temperaturen von 100-160°C in Gegenwart von Natrium-C₁-C₆-alkylat und Alkohol; und (3) Gewinnen des kondensierten Produktes.

2. Verfahren nach Anspruch 1, wobei ein gemischtes Methyl/Ethyl-Dialkylsuccinylsuccinat unter Verwendung von Ethanol in Schritt (1) hergestellt wird.

3. Verfahren nach Anspruch 1, wobei der Katalysator ein Metallalkoholat ist.

4. Verfahren nach Anspruch 1, wobei Schritt (1) bei Umgebungstemperaturen bis 100°C stattfindet.

5. Verfahren nach Anspruch 1, wobei in Schritt (1) das Alkanol in einer Menge von 1 bis 10 Mol pro Mol Dimethylsuccinat vorliegt und der Katalysator in einer Menge von 0,01-5 Gewichtsprozent Bernsteinsäuredimethylester vorliegt.

6. Verfahren nach Anspruch 1, wobei ein von dem Alkanol verschiedenes organisches Lösungsmittel während der Schritte (1) und (2) vorliegt.

7. Verfahren nach Anspruch 1, wobei in Schritt (2) das Natriumalkylat in einer Menge von 1 bis 3 Mol vorliegt und der Alkohol in einer Menge von 1 bis 10 Mol vorliegt, jeweils pro Mol des gemischten Dialkylsuccinats.

8. Verfahren nach Anspruch 1, wobei Schritte (1) und (2) in-situ durchgeführt werden.

9. Verfahren zur Herstellung von Methyl/Ethyl- oder Methyl/Isobutyl - Dialkyl- 2,5 -diarylamino- 3,6-dihydroterephthalsäureestern, umfassend Kondensieren des aus Schritt (2) des Verfahrens von Anspruch 1 erhaltenen gemischten Methyl/Ethyl- oder Methyl/Isobutyl-Dialkylsuccinylsuccinats mit einem Überschuß von einem Arylamin.

10. Verfahren nach Anspruch 6, wobei das zusätzliche Lösungsmittel ein Gemisch von (a) Diphenylether oder einem oder mehreren alkylierten Diphenylethern oder ein Gemisch davon darstellt und (b) Biphenyl oder eines oder mehrere alkylierte Biphenyle oder ein Gemisch davon darstellt.

## Revendications

1. Procédé pour la préparation de succinylsuccinates de dialkyles mixtes méthyl/éthyle ou méthyl/isobutyle qui comprend les étapes de (1) transestérification du succinate de diméthyle avec l'éthanol ou l'isobutanol en présence d'un catalyseur de transestérification ; (2) condensation de deux moles de succinate de dialkyle mixte de l'étape (1) sur le succinylsuccinate de dialkyle mixte méthyl/éthyle ou méthyl/isobutyle ou leur sel disodique, à des températures de 100 à 160°C, en présence d'alkylate en C₁-C₆ de sodium et d'alcool ; et (3) récupération du produit condensé.

2. Procédé de la revendication 1, où on prépare un succinylsuccinate de dialkyle mixte méthyl/éthyle en utilisant de l'éthanol à l'étape (1).

3. Procédé de la revendication 1, où ledit catalyseur est un alcoolate de métal.

4. Procédé de la revendication 1, où ladite étape (1) est effectuée à des températures allant de la température ambiante à 100°C.

5. Procédé de la revendication 1, où dans l'étape (1) ledit alcanol est présent dans une quantité de 1 à 10 moles par mole de succinate de diméthyle et ledit catalyseur est présent dans une quantité de 0,01 à 5% en poids de succinate de diméthyle.

6. Procédé de la revendication 1, où un solvant organique différets dudit alcanol est présent dans les étapes (1) et (2).

7. Procédé de la revendication 1, où dans l'étape (2), l'akylate de sodium est présent dans une quantité de 1 à 3 moles et l'alcool est présent dans une quantité de 1 à 10 moles, chacun par mole de succinate de dialkyle mixte.

8. Procédé de la revendication 1, où les étapes (1) et (2) sont effectuées in situ.

9. Procédé pour la préparation d'esters mixtes de 2,5-diarylamino-3,6-dihydrotéréphtalate de dialkyle méthyl/éthyle ou méthyl/isobutyle qui comprend la condensation du succinylsuccinate de dialkyle mixte méthyl/éthyle ou méthyl/isobutyle résultant de l'étape (2) du procédé de la revendication 1, avec un excès en arylamine.

10. Procédé de la revendication 6, où le solvant supplémentaire est un mélange (a) d'éther diphénylique ou d'un ou plusieurs éthers diphényliques alkylés ou de leur mélange, et (b) de biphényle ou d'un ou plusieurs biphényles alkylés ou de leur mélange.
